# EUROPÄISCHE PATENTSCHRIFT

(11) **EP 1 307 241 B1**
(45) Veröffentlichungstag und Bekanntmachung des Hinweises auf die Patenterteilung: **25.05.2005**
(21) Anmeldenummer: 01957701.4
(22) Anmeldetag: 04.07.2001
(51) Int. Cl.: A61L 2/00, A61L 2/10

(54) **PHOTODYNAMISCHE BEHANDLUNG UND UV-B- BESTRAHLUNG EINER THROMBOZYTEN-SUSPENSION**
PHOTODYNAMIC TREATMENT AND UV-B IRRADIATION OF A THROMBOCYTE SUSPENSION
TRAITEMENT PHOTODYNAMIQUE ET EXPOSITION AUX RAYONS U.V.-B D'UNE SUSPENSION DE THROMBOCYTES

(30) Priorität: 04.07.2000 DE 10031851
(43) Veröffentlichungstag der Anmeldung: 07.05.2003
(73) Patentinhaber: BLUTSPENDEDIENST DER LANDESVERBÄNDE DES DRK NIEDERSACHSEN, SACHSEN-ANHALT, THÜRINGEN, OLDENBURG UND BREMEN GGMBH, 31830 Springe (DE)
(72) Erfinder: MOHR, Harald, 30177 Hannover (DE); REDECKER-KLEIN, Anette, D-31708 Ahnsen (DE)
(74) Vertreter: Schupfner, Georg U.
(86) Internationale Anmeldenummer: PCT/DE2001/002410
(87) Internationale Veröffentlichungsnummer: WO 2002/002152

(56) Entgegenhaltungen:
- WO-A-00/04930
- WO-A-96/08965
- WO-A-97/03706
- WO-A-98/31219
- DE-A- 3 930 510
- US-A- 5 545 516
- US-A- 6 077 659

## Beschreibung

Die Erfindung betrifft ein Verfahren zur Inaktivierung von Viren und zum Abtöten von Leukozyten in Thrombozyten-Suspensionen durch eine Kombination von photodynamischer Behandlung und UV-B-Bestrahlung.

Es ist bekannt, dass die therapeutische Anwendung von Blutpräparaten das Risiko birgt, dass die Empfänger des Blutpräparates mit Viren infiziert werden. Genannt seien z.B. die Viren Hepatitis-Viren B (HBV) und C (HCV) sowie die Aids-Erreger HIV-1 und HIV-2. Das Risiko besteht immer dann, wenn bei der Herstellung des Präparates kein Schritt zur Virusinaktivierung bzw. Viruseliminierung Anwendung findet.

Bei gereinigten Plasmaproteinkonzentraten wie z.B. Albumin, Faktor-VIII- und Faktor-IX-Präparaten werden Verfahren zur Virusinaktivierung bzw. Viruseliminierung angewandt, so dass diese inzwischen als virussicher gelten. Auch das Virusrisiko von Frischplasma lässt sich durch Anwendung unterschiedlicher Methoden zumindest reduzieren. Eine Methode ist z.B. die Quarantänelagerung. Hierbei wird das Plasma für 3 bis 6 Monate tiefgefroren gelagert und erst dann zur Anwendung freigegeben, wenn eine neue Blutprobe des betreffenden Spenders erneut auf die üblichen Marker für HBV, HCV, HIV-1 und HIV-2 geprüft und als negativ befunden wurde. Eine derartige Methode ist für zelluläre Blutprodukte, wie z.B. E-rythrozyten- und Thrombozytenkonzentrate nicht anwendbar, da diese nur eine Haltbarkeitsdauer von ca. 7 Wochen bzw. 5 Tagen besitzen. Zelluläre Blutprodukte können aus naheliegenden Gründen auch nicht durch eine Solvent/Detergens-Behandlung virussicher gemacht werden, wie dies bei Plasmaprotein-Konzentraten und auch bei Plasma möglich ist: Erythrozyten und Thrombozyten würden hierdurch lysiert.

Es wird intensiv daran gearbeitet, zelluläre Blutprodukte mittels photodynamischer Verfahren zu dekontaminieren. Die photodynamische Virusinaktivierung beruht darauf, dass das betreffende Präparat in Lösung bzw. Suspension in Gegenwart einer photoaktiven Substanz, eines Photosensitizers, belichtet wird. Das eingestrahlte Licht muss eine Wellenlänge aufweisen, die von der photoaktiven Substanz absorbiert wird. Er wird dadurch aktiviert und überträgt diese Aktivierungsenergie entweder direkt auf ein Substrat, das dadurch zerstört bzw. geschädigt wird oder aber auf Sauerstoffmoleküle: Aktivierte Sauerstoff-Spezies, d.h. Sauerstoffradikale oder Singlet-Sauerstoff wirken stark viruzid. Im günstigen Fall besitzt die eingesetzte photoaktive Substanz eine große Affinität zu Virusbestandteilen, z.B. zur viralen Nukleinsäure und nur eine geringe zu sonstigen Komponenten, die im betreffenden Präparat enthalten sind. So werden Viren inaktiviert und andere Komponenten nicht alteriert. Breitere Verwendung findet derzeit nur ein photodynamisches Verfahren gemäß dem Europäischen Patent 0 491 757-B1 (H. Mohr und B. Lambrecht, Verfahren zur Inaktivierung von Viren in Blut und Blutprodukten). Es wird zur Virusinaktivierung bei Frischplasma eingesetzt. Als photoaktive Substanz dient in der technischen Anwendung vor allem der Phenothiazin-Farbstoff Methylenblau. Anstelle von Methylenblau kann auch Toluidinblau verwendet werden. Auch die Demethylierungsprodukte von Methylenblau, d.h. die Azurfarbstoffe A, B und C sowie Thionin sind photodynamisch aktiv und zur photodynamischen Virusinaktivierung geeignet.

Das U.S.-Patent 5,545,516 (S. J. Wagner: Inactivation of extracellular enveloped viruses in blood and blood components by phenthiazin-5-ium dyes plus light) beschreibt die Inaktivierung extrazellulärer Viren mit Hilfe von Phenothiazinfarbstoffen in Kombination mit sichtbarem Licht. Nach der US 5,545,516 werden die Präparate vor der photodynamischen Behandlung mittels spezieller Filter von Leukozyten befreit, da die Virusinaktivierungsmethode keine zellassozierten Viren bzw. Proviren erfasst. Die Methode ist gleichfalls nicht imstande, die im Blut vorkommenden kleinen nicht umhüllten Viren, wie z.B. Hepatits-A-Viren (HAV) zu inaktivieren. Freie Viren, die Lipidhüllen besitzen, wie z.B. der Aids-Erreger HIV-1 und die Hepatits-Viren B und C (HBV, HCV), sind dagegen gemäß diesem Verfahren inaktivierbar. *Auch aus der WO 00*/*04930 und WO 96*/*08965 sind Verfahren zur Inaktivierung von Pathogenen in biologischen Proben bekannt, die photoaktive Substanzen einsetzen, die u.a. im UV-A-Bereich absorbieren und mit Strahlung im Wellenlängenbereich von UV-A bis in den sichtbaren Bereich aktiviert werden*.

Leukozyten in Blutprodukten können mittels UV-Bestrahlung abgetötet werden. Bei Thrombozytensuspensionen hat sich hierfür die Bestrahlung mit UV-B (Wellenlängenbereich 290-320 mn) als sinnvoll erwiesen, da zur Leukozytendepletion ein Energieeintrag von 1 bis 3 J/cm² in der Regel ausreicht, der die Thrombozyten noch nicht all zu sehr beeinträchtigt, so dass sie therapeutisch einsetzbar bleiben. Ein Energie-Eintrag durch Betrahlung mit UV-B von größer 10 J/cm² wirkt zusätzlich viruzid (K.N. Prodoux; J.C. Fratanoni, E.J. Boone und R.F. Bonner in Blood, 70 (2), 589-592 (1987): Use of Laser-UV for inactivation of virus in blood products. Allerdings werden Thrombozyten hierbei derart geschädigt, dass ihre Anwendbarkeit in Frage gestellt werden muss (J.C. Fratanoni und K.N. Prodoux in Transfusion 30(6); 480-481 (1990); Viral inactivation of blood products).

Aufgabe der Erfindung ist es somit, eine effektive Methode zur Inaktivierung humanpathogener Viren und von Leukozyten in Thrombozyten-Suspensionen, insbesondere Thrombozytenkonzentraten (TK), zur Verfügung zu stellen. TK werden aus Blutspenden durch Differentialzentrifugation oder direkt von Spendern durch maschinelle Trombozytapherese gewonnen.

Überraschend wurde gefunden, dass die Kombination von photodynamischer Behandlung mit einer UV-B-Bestrahlung in Thrombozyten-Suspensionen bzw. TK effektiv die der photodynamischen Virusinaktivierung zugänglichen Viren erfaßt und gleichzeitig die in den Medien enthaltenen Leukozyten abzutöten vermag und somit das Infektionsrisiko durch zellassoziierte Viren bzw. Proviren beseitigt. Weiterhin wurde überraschenderweise gefunden, dass durch die Kombination der Methoden die zum Abtöten der Leukozyten erforderliche Menge an UV-B-Strahlung bedeutend geringer sein kann als bei der Bestrahlung mit UV-B allein. Gleichfalls überraschend war, dass die zusätzliche Behandlung der Thrombozytensuspensionen mit UV-B mit einer Intensität, die für sich allein fast wirkungslos bei der Inaktivierung von Viren ist, die Effizienz der photodynamischen Behandlung deutlich steigert.

Das erfindungsgemäße Verfahren zur Behandlung einer Thrombozyten-Suspension ist wie folgt gekennzeichnet:
(A) Aussetzen der Suspension einer Strahlung eines Wellenlängenbereiches von 400 bis 750 nm, vorzugsweise 550 bis 700 nm, in Gegenwart einer oder mehrer photoaktiver Substanzen, welche in dem Wellenlängenbereich ein oder mehrere Absoptionsmaxima aufweisen, und
(B) Aussetzen der Suspension einer Strahlung eines Wellenlängenbereiches von 270 bis 320 nm, vorzugsweise 290 bis 320 nm mit einem Energieeintrag von 0,1 bis 10 J/cm².
wobei in Schritt (B) keine im Wellenlängenbereich gemäß Schritt (B) photoaktivierbare Substanz anwesend ist und die Schritte (A) und (B) in der Abfolge erst (A) dann (B) angewandt werden. Bevorzugte Ausführungsformen sind Gegenstand der Unteransprüche.

Die Thrombozyten-Suspension weist vorzugsweise eine Konzentration von mehr 5x10⁸ Thrombozyten pro ml, besonders bevorzugt von mehr als 10⁹/ml auf. Die Thrombozyten können z.B. in Plasma oder in einem Thrombozyten-Lagerungsmedium mit beliebigem Plasma-Gehalt suspendiert sein.

Schritt A beinhaltet eine photodynamische Behandlung der Thrombozyten-Suspension in Gegenwart einer photoaktiven Substanz mit sichtbarem Licht; Schritt B beinhaltet die Bestrahlung des Präparats - der thrombozytenhaltigen Suspension - mit Licht im Wellenlängenbereich von UV-B. Als UV-B Bereich der Bestrahlung im Sinne der Erfindung wird der Wellenlängenbereich 270 nm bis 330 nm angesehen.

Die Konzentration der eingesetzten photoaktiven Substanz und der Energie-Eintrag durch die Belichtung und die UV-B-Bestrahlung sind so bemessen, dass eventuell vorhandene Viren inaktiviert und die in den Thrombozyten-Suspensionen enthaltenen Leukozyten abgetötet werden, die Funktionsfähigkeit der Thrombozyten aber erhalten bleibt.

Als Behältnisse zur Behandlung der Thrombozyten-Suspensionen dienen UV-Bdurchlässige Behältnisse, die vorzugsweise aus Kunststoff bestehen und z.B. die Form von Beuteln haben können. Es ist aber auch denkbar, die photodynamische und die UV-B-Behandlung in unterschiedlichen Behältnissen durchzuführen.

Denkbar ist auch, die UV-B-Behandlung der Thrombozyten-Suspensionen durchzuführen, während die Thrombozyten-Suspensionen von einem Behältnis in ein anderes überführt wird.

Als photoaktive Substanzen können z.B. die Phenothiazin-Farbstoffe Methylenblau, Azur A, B, C und Thionin benutzt werden. Andere photoaktive Substanzen sind, z.B. in den für die Virusinaktivierung in Blutprodukten aus der Literatur bekannten Konzentrationen, ebenfalls vorteilhaft einsetzbar. Bei Phenothiazin-Farbstoffen wie Thionin liegt der denkbare Konzentrationsbereich zwischen ca. 0,1 und 10 µM, bevorzugt zwischen ca. 0,5 und 5 µM oder 1 und 5 µM.

Als Lichtquelle für die photodynamische Behandlung, insbesondere beim Einsatz von Thionin, dienen vorzugsweise Niederdruck-Natriumdampflampen, deren Lichtemissionsmaximum bei 590 nm liegt. Dies entspricht in etwa dem Absorptionsmaximum von Thionin, das in wässriger Lösung ca. 595 nm beträgt. Es sind aber auch andere Lichtquellen denkbar, insbesondere dann, wenn eine photoaktive Substanz verwendet wird, die Licht in einem anderen Wellenlängenbereich absorbiert als beispielsweise Thionin.

Für die Bestrahlung mit UV-B können spezielle Röhren, Lampen oder Laser eingesetzt werden, die ultraviolettes Licht im Wellenlängenbereich zwischen ca. 270 - 330 nm emittieren. Der Energie-Eintrag durch die UV-B-Behandlung kann zwischen 0,1 und 10 J/cm², vorzugsweise zwischen 0,3 und 6 J/cm², besonders bevorzugt zwischen 0,5 und 3 J/cm² liegen.

### Versuchsbeispiele

### 1. Allgemeines:

Die nachstehend beschriebenen Versuche wurden mit TK durchgeführt, die aus einzelnen Blutspenden isoliert wurden und in Blutplasma suspendiert waren. Als photoaktive Substanz wurde Thionin (Th) eingesetzt. Ähnliche Ergebnisse lassen sich aber auch mit anderen photoaktiven Substanzen erzielen, beispielsweise den Phenothiazin-Farbstoffen Methylenblau und dessen Abkömmlingen Azur A, B und C. Die Erfindung wird durch die Versuchsbeispiele lediglich erläutert, nicht aber in ihrem Umfang beschränkt.

### 2. Materialien und Methoden

Die bei den Versuchen eingesetzten TK wurden in Thrombozytenrotatoren für bis zu 5 Tage gelagert. Lagerbehältnisse waren kommerziell erhältliche PVC-Beutel. Zur photodynamischen und zur UV-B-Behandlung wurden die TK in Plastikbeutel aus Poly-Olefin überführt, deren Folienmaterial durchlässig für UV-B ist. Zur Belichtung in der Gegenwart von Thionin wurde eine Anlage benutzt, die mit Niederdruck-Natrium-Dampflampen ausgestattet ist. Die TK wurden von beiden Seiten belichtet. Zur Bestrahlung mit UV-B wurde ein Flächenstrahler eingesetzt, der mit UV-Röhren versehen war, die vornehmlich UV-Licht im Wellenlängenbereich zwischen 290 und 320 nm emittierten.

Als Testvirus wurde im allgemeinen Vesicular Stomatitis-Virus (VSV) eingesetzt, das leicht in der Zellkultur propagierbar und demzufolge auch in CPE-Assays quantifizierbar ist (CPE = cytopatischer Effekt). Im Versuch 1 wurden darüber hinaus noch eine Reihe anderer Viren eingesetzt. VSV wurde in Vero-Zellen propagiert. Die selben Zellen wurden auch für die Infektiösitätsassays eingesetzt, mit denen die Virustiter bestimmt wurden. Das benutzte Zellkulturmedium war RPMI 1640 mit 10 % fötalem Kälberserum und Antibiotika. Die Assays wurden in Mikrotiterplatten durchgeführt. Die betreffenden Proben wurden in 1 zu 3er-Schritten herunterverdünnt. Pro Verdünnung wurden 8 Replikate getestet. Die Virustiter sind als log₁₀TCID₅₀ ausgedrückt (TCID = Tissue Culture Infective Doses) und wurden entsprechend der Vorgabe von Kärber und Spearman berechnet (G. Kärber in Naunym-Schmiedebers Arch. Exp. Pathol. Pharmakol. 162, 480-483 (1931): Beitrag zur kollektiven Behandlung pharmakologischer Reihenversuche; C. Spearman in Br.J.Psychol. 2, 277-282 (1908): The method of "right and wrong cases" ("constant stimuli") without Gauss for mulae).

Als Funktionstests für Thrombozyten wurden die hypotone Schockreaktion und die Collagen-induzierte Aggregation verwendet.

Mononukleäre Zellen wurden mittels Dichtegradienten-Zentrifugation aus Spenderblut isoliert. Bei den betreffenden Experimenten wurden diese in einer Konzentration von 5 x 10⁵/ml den Thrombozytensuspensionen zugesetzt. Nach der photodynamischen Behandlung bzw. UV-B-Bestrahlung wurden Aliquots der Suspensionen niedrigtourig zentrifugiert (1500 rpm für 4 min.). Die pelettierten Zellen wurden dreimal mit Zellkulturmedium gewaschen (RPMI 1640 mit 10 % fötalem Kälberserum und Antibiotika) und dann im selben Medium resuspendiert. Die Zellkonzentration wurde auf 5x10⁵/ml eingestellt. Für den Proliferationsassay wurden die Zellen mit Concanavalin A (ConA, 2 µg/ml) stimuliert und in 200 µl-Aliquots für 3 - 4 Tage bei 37 °C im CO₂-begasten Brutschrank kultiviert. Dann wurde den Zellkulturen zugegeben. Vier Stunden später wurde die BRDU-Einbaurate (BRDU = Brom-Deoxyuridin) spektralphotometrisch bei einer Wellenlänge von 450 nm (OD₄₅₀) bestimmt. Die Extinktionswerte sind dem BRDU-Einbau und damit der Viabilität der Zellen proportional.

### Versuch 1: Inaktivierung von Viren in TK durch Behandlung mit Thionin/Licht.

Eine Reihe von Viren wurde daraufhin untersucht, ob und in welchem Ausmaß sie durch die Behandlung mit Thionin/Licht inaktivierbar sind. Die Konzentration der photoaktiven Substanz war 1 µM. Wie die in der Tab. 1 zusammengefassten Ergebnisse zeigen, erwiesen sich verschiedene Viren als unterschiedlich empfindlich: so waren die Modellviren für das menschliche Hepatitis-C-Virus BVDV und CSFV sowie das Togavirus SFV nach 5 Minuten Belichtung bereits völlig inaktiviert, während die Infektiösität von VSV und von SV-40 auch nach 30 Minuten noch nicht vollständig beseitigt worden war.

### Versuch 2: Inaktivierung von VSV in TK durch Bestrahlung mit UV-B

Wie aus der Tab. 2 hervorgeht, ist VSV sehr resistent gegenüber der Bestrahlung mit UV-B. Noch nach 60 min. bzw. einem Energie-Eintrag von ca. 20 J/cm² war das Virus noch nicht völlig inaktiviert. Ab ca. 10 min. bzw. 3 J/cm² wirkte sich demgegenüber die UV-B-Bestrahlung negativ auf Funktionen und Lagerfähigkeit von Thrombozyten aus (nicht gezeigt).

**Tab. 1:**

| *Photodynamische Inaktivierung von Viren in TK durch Thionin*/*Lichtbehandlung. VSV = Vesicular Stomatitis-Virus; CSFV = Classical Swine Fever-Virus); BVDV = Bovines Virales Diarrhoe-Virus; SFV = Semliki Forest-Virus; HIV-1 = Humanes Immundefizienz-Virus, Typ 1; SHV-1 = Suid Herpes-Virus, Typ 1; SV-40 = Simian-Virus 40; ssRNA = Single Strand RNA; dsDNA = Double Strand DNA , * Reduktion des Virustiters in log*_{*10*}*TCID*_{*50*}*.* | | | | |
|---|---|---|---|---|
| Virus | **VSV** | **CSFV** | **BVDV** | **SFV** |
| Familie | Rhabdo | Flavi | Flavi | Toga |
| Genom | ssRNA | ssRNA | ssRNA | ssRNA |
| Belichtungszeit (min) | 30 | 5 | 5 | 5 |
| Reduktion des Virustiters | 4,4 | ≥5,5 | ≥4,9 | ≥5,2 |

| Virus | **HIV-1** | **SHV-1** | **SV-40** | |
|---|---|---|---|---|
| Familie | Retro | Herpes | Papova | |
| Genom | ssRNA | dsDNA | DsDNA | |
| Belichtungszeit (min) | 30 | 10 | 30 | |
| Reduktion des Virustiters* | ≥5,7 | ≥3,6 | 3,9 | |

**Tab. 2:**

| *Inaktivierung von VSV in Thrombozytenkonzentraten durch Bestrahlung mit UV-B* | | | |
|---|---|---|---|
| **UV-B** | | **Virus-Titer** (log₁₀TCID₅₀) | **Reduktionsfaktor** (log₁₀TCID₅₀) |
| Min. | J/cm² | | |
| 0 | 0 | 6,44 | 0 |
| 10 | 3,25 | 5,48 | 0,96 |
| 20 | 6,5 | 4,53 | 1,91 |
| 30 | 9,75 | 4,35 | 2,09 |
| 40 | 13 | 3,28 | 3,16 |
| 50 | 16,25 | 2,33 | 4,11 |
| 60 | 19,5 | 1,61 | 4,83 |

### Versuch 3: Inaktivierung von VSV in TK durch die Kombination Thionin / Licht und UV-B Strahlung

Bei diesen Versuchen betrug die Konzentration von Thionin wiederum 1 µM und die Belichtungszeit 30 min. Der Energie-Eintrag durch UV-B Strahlung betrug 2,4 J/cm² (Bestrahlungszeit: 8 min). Durch die photodynamische Behandlung allein wurde die Infektiösität um 4 bzw. 4,42 log₁₀ reduziert, durch UV-B Strahlung allein um 1,97 bzw. 2,21 log₁₀. In der Kombination wurde im ersten Versuch die Infektiösität völlig beseitigt (≥ 7,04 log₁₀), im zweiten um 6,26 log₁₀ reduziert (Tab. 3).

**Tab. 3:**

| *Inaktivierung von VSV in TK durch Thionin*/*Licht, UV-B und die Kombination beider Arbeitsschritte.* | | | |
|---|---|---|---|
| **Thionin/Licht** | **UV-B** | **Infektiösität (log**_{**10**}**LTCID**_{**50**}**)** | |
| | | **Versuch 1** | **Versuch 2** |
| - | - | 7,28 ± 0,29 | 6,68 ± 0,21 |
| + | - | 2,86 ± 0,31 | 2,68 ± 0,12 |
| - | + | 5,07 ± 0,12 | 4,71 ± 0,17 |
| + | + | ≤0,24 ± 0,00 | 0,42 ± 0,21 |

### Versuch 4: Einfluss der Behandlung mit Thionin / Licht in Kombination mit UV-B auf die Thrombozytenfunktionen.

Wie die Tab. 4 und 5 zeigen, werden sowohl die HSR (Hypotone Schockreaktion) als auch die Collagen-induzierte Aggregation von TK durch die kombinierte Behandlung mit Thionin/Licht und UV-B (Versuchsbedingungen: s. Versuch 3) nicht wesentlich stärker beeinflusst als durch die photodynamische Behandlung allein.

**Tab. 4:**

| | | | | | | | |
|---|---|---|---|---|---|---|---|
| *Einfluss der Behandlung von Thrombozytensuspensionen mit Thionin*/*Licht ± UV-B auf die HSR (in % ausgedrückt) gemessen am Tag 1 und am Tag 3 nach der Behandlung.* | | | | | | | |

| **Nr.** | **Behandlung** | **Versuch 1** | | **Versuch 2** | | **Versuch 3** | |
|---|---|---|---|---|---|---|---|
| | | Tag 1 | Tag 3 | Tag 1 | Tag 3 | Tag 1 | Tag 3 |
| 1 | Kontrolle | 71,98 | 63,07 | 66,71 | 60,78 | 78,16 | 62,59 |
| 2 | THIONIN/Licht | 65,49 | 49,16 | 56,24 | 52,61 | 69,30 | 55,49 |
| 3 | UV-B (2,4 J/cm²) | 61,06 | 57,09 | 56,26 | 48,80 | 65,67 | 52,49 |
| 4 | Thionin/Licht+UV-B | 47,86 | 51,16 | 42,37 | 30,26 | 54,45 | 48,31 |

**Tab. 5:**

| *Einfluss der Behandlung von Thrombozytensuspensionen mit Thionin* / *Licht ± UVB auf die Collagen-induzierte Aggregation (in %, ausgedrückt), gemessen am Tag 1 und am Tag 3 nach der Behandlung.* | | | | | | | |
|---|---|---|---|---|---|---|---|
| **Nr.** | **Behandlung** | **Versuch 1** | | **Versuch 2** | | **Versuch 3** | |
| | | Tag 1 | Tag 3 | Tag 1 | Tag 3 | Tag 1 | Tag 3 |
| 1 | Kontrolle | 88,00 | 23,00 | 83,33 | 30,00 | 79,67 | 30,33 |
| 2 | THIONIN/Licht | 73,25 | 13,75 | 84,67 | 27,67 | 69,67 | 15,67 |
| 3 | UV-B (2,4 J/cm²) | 76,25 | 11,25 | 73,33 | 24,50 | 75,67 | 20,00 |
| 4 | Thionin/Licht+UV-B | 69,75 | 16,75 | 76,67 | 53,50 | 58,33 | 30,33 |

### Versuch 5: Inaktivierung von T-Lymphozyten in TK durch UV-B; Einfluss von Thionin/Licht.

TK wurden mononukleäre Zellen in einer Konzentration von 5x10⁵/ml zugefügt; dann wurden sie für verschiedene Zeiten mit UV-B bestrahlt bzw. allein oder zusätzlich mit Thionin/Licht behandelt (Farbstoff-Konzentration: 2 µM; Belichtungsdauer: 30 min.). Wie die in der Tab. 6 zusammengestellten Ergebnisse zeigen, war zur völligen Inaktivierung der Zellen eine Bestrahlungszeit von mindestens 4 min. (1,2 J/cm²) notwendig. Wurden die TK zusätzlich mit Thionin/Licht behandelt, konnte sie auf ca. 3 min. verkürzt werden, obwohl Thionin/Licht allein keinen Einfluss auf die Proliferation der Zellen hatte.

**Tab. 6:**

| *Inaktivierung von T-Lymphozyten in Thrombozytenkonzentraten durch Bestrahlung mit UV-B. Verstärkung der Wirkung durch vorherige Behandlung mit Th*/*Licht für 30 min. Die Zellen wurden nach der Bestrahlung bzw. Th*/*Licht- Behandlung mit ConA stimuliert. Die OD*_{*450nm*}*-Werte sind Mittelwerte aus Dreifachbestimmungen. Sie repräsentieren die Einbaurate von BRDU in die Zellen nach einer Kultivierungszeit von 3 Tagen.* | | | | | |
|---|---|---|---|---|---|
| **Nr.** | **UV-B** **(J/*cm***^{**2**}**)** | **Th/Licht** | **ConA-Aktivierung** | **Absorption OD**_{**450nm**} | **Bemerkungen** |
| 1 | 0 | 0 | - | 0,276 | Negativ-Kontrolle |
| 2 | 0 | 0 | + | 2,269 | Positiv-Kontrolle |
| 3 | 0,6 | - | + | 1,767 | |
| 4 | 0,9 | - | + | 0,747 | |
| 5 | 1,2 | - | + | 0,297 | |
| 6 | 0,6 | + | + | 1,020 | |
| 7 | 0,9 | + | + | 0,387 | |
| 8 | 1,2 | + | + | 0,240 | |

## Patentansprüche

1. Verfahren zur Behandlung einer Thrombozyten-Suspension umfassend die folgenden Schritte :
(A) Aussetzen der Suspension einer Strahlung eines Wellenlängenbereichs von 400 bis 750 nm in Gegenwart einer oder mehrer photoaktiver Substanzen, welche in dem Wellenlängenbereich ein oder mehrere Absoptionsmaxima aufweisen, und
(B) Aussetzen der Suspension einer Strahlung eines Wellenlängenbereiches von 270 bis 320 nm mit einem Energieeintrag von 0,1 bis 10 J/cm²,
wobei
- die Schritte (A) und (B) in der Abfolge erst (A) dann (B) angewandt werden und
- in Schritt (B) keine photoaktive Substanz anwesend ist, die ein Absorptionsmaximum im Wellenlängenbereich gemäß Schritt (B) aufweist.

2. Verfahren gemäß Anspruch 1, **dadurch gekennzeichnet, dass** die photoaktive Substanz ein Phenothiazin-Farbstoff ist.

3. Verfahren gemäß Anspruch 2, **dadurch gekennzeichnet, dass** als Phenothiazin-Farbstoffe Thionin, Methylenblau, Toluidinblau und/oder die Azurfarbstoffe A, B und C eingesetzt werden.

4. Verfahren gemäß einem der Ansprüche 2 oder 3, **dadurch gekennzeichnet, dass** die photoaktive Substanz in Schritt (A) in einer Konzentration von 0,5 bis 10 µM, vorzugsweise 0,5 bis 5 µM, eingesetzt wird.

5. Verfahren gemäß einem der vorhergehenden Ansprüche, **dadurch gekennzeichnet, dass** die Intensität der Strahlung im Schritt (B) so bemessen ist, dass die Proliferationsfähigkeit von T-Lymphozyten, die in den Thrombozytenkonzentraten enthalten sind, um wenigstens 50 % reduziert wird, vorzugsweise um mehr als 75 %.

6. Verfahren gemäß einem der vorhergehenden Ansprüche, **dadurch gekennzeichnet, dass** in Schritt (B) der Energieeintrag 0,5 bis 3 J/cm² beträgt.

7. Verfahren gemäß einem der vorhergehenden Ansprüche, **dadurch gekennzeichnet, dass** in Schritt (B) die Suspension einer Strahlung eines Wellenlängenbereiches von 290 bis 320 nm ausgesetzt wird.

8. Verfahren gemäß einem der vorhergehenden Ansprüche, **dadurch gekennzeichnet, dass** die Thrombozyten-Suspensionen Thrombozytenkonzentrate sind.

9. Verfahren gemäß einem der vorhergehenden Ansprüche, **dadurch gekennzeichnet, dass** die Thrombozyten-Suspensionen bzw. Thrombozytenkonzentrate aus Blutspenden oder durch Thrombozytapherese gewonnen wurden.

10. Verfahren gemäß einem der vorhergehenden Ansprüche, **dadurch gekennzeichnet, dass** die Behandlung der Suspension gemäß Schritt (A) und/oder Schritt (B) in für die jeweilige Strahlung durchlässigen Kunststoffbehältnissen erfolgt.

11. Verfahren gemäß einem der vorhergehenden Ansprüche, **dadurch gekennzeichnet, dass** die Behandlung der Suspension gemäß Schritt (A) und/oder Schritt (B) in einer für die jeweilige Strahlung durchlässigen Durchfluss-Apparatur erfolgt.

12. Verfahren gemäß einem der vorhergehenden Ansprüche , **dadurch gekennzeichnet, dass** die Viruskonzentration der Suspension um mindestens 5, vorzugsweise um mindestens 6 log₁₀ Stufen erniedrigt wird.

## Claims

1. A process for the treatment of a thrombocyte suspension comprising the following steps:
(A) exposure of the suspension to radiation in a wavelength range of 400 to 750 nm in the presence of one or several photoactive substances which have one or several absorption maxima in the wavelength range, and
(B) exposure of the suspension to radiation in a wavelength range of 270 to 330 nm, with an energy input of 0.1 to 10 J/cm²
wherein
- steps (A) and (B) are applied in the order first (A) then (B) and
- in step (B) there is no photoactive substance present which comprises an absorption maximum in the wavelength range of the radiation according to step (B).

2. The process according to claim 1, **characterised in that** the photoactive substance is a phenothiazine dye.

3. The process according to claim 2, **characterised in that** thionine, methylene blue, toluidine blue and/or azure A, azure B and azure C are used as phenothiazine dyes.

4. The process according to one of claims 2 or 3, **characterised in that** the photoactive substance in step (A) is used in a concentration of 0.5 to 10 µM, preferably 0.5 to 5 µM.

5. The process according to one of the preceding claims, **characterised in that** the intensity of the radiation in step (B) is set such that the proliferation capability of T-lymphocytes contained in the thrombocyte concentrates is reduced by at least 50 %, preferably by more than 75 %.

6. The process according to one of the preceding claims, **characterised in that** in step (B) the energy input is 0.5 to 3 J/cm².

7. The process according to one of the preceding claims, **characterised in that** in step (B) the suspension is exposed to radiation in a wavelength range of 290 to 320 nm.

8. The process according to one of the preceding claims, **characterised in that** the thrombocyte suspensions are thrombocyte concentrates.

9. The process according to one of the preceding claims, **characterised in that** the thrombocyte suspensions or thrombocyte concentrates were obtained from blood donation or by thrombocyte apheresis.

10. The process according to one of the preceding claims, **characterised in that** the treatment of the suspension in accordance with step (A) and/or step (B) takes place in plastic containers transparent to the appropriate radiation.

11. The process according to one of the preceding claims, **characterised in that** the treatment of the suspension in accordance with step (A) and/or step (B) takes place in a flow-through apparatus transparent to the appropriate radiation.

12. The process according to one of the preceding claims, **characterised in that** the virus concentration of the suspension is reduced by at least 5 log₁₀, preferably by at least 6 log₁₀ steps.

## Revendications

1. Procédé pour le traitement d'une suspension de thrombocytes comprenant les étapes suivantes :
(A) soumission de la suspension à un rayonnement présentant une plage de longueurs d'onde de 400 à 750 nm en présence d'une ou de plusieurs substances photoactives, qui présentent un ou plusieurs maxima d'absorption dans la plage de longueurs d'onde et
(B) soumission de la suspension à un rayonnement présentant une plage de longueurs d'onde de 270 à 320 nm avec une introduction d'énergie de 0,1 à 10 J/cm²,
où
- les étapes (A) et (B) sont réalisées dans l'ordre (A), puis (B) et
- il n'y a pas de substance photoactive dans l'étape (B) qui présente un maximum d'absorption dans la plage de longueurs d'onde selon l'étape (B).

2. Procédé selon la revendication 1, **caractérisé en ce que** la substance photoactive est un colorant de phénothiazine.

3. Procédé selon la revendication 2, **caractérisé en ce qu'**on utilise comme colorants de phénothiazine la thionine, le bleu de méthylène, le bleu de toluidine et/ou les colorants Azur A, B et C.

4. Procédé selon la revendication 2 ou 3, **caractérisé en ce que** la substance photoactive dans l'étape (A) est utilisée en une concentration de 0,5 à 10 µM, de préférence de 0,5 à 5 µM.

5. Procédé selon l'une quelconque des revendications précédentes, **caractérisé en ce que** l'intensité du rayonnement dans l'étape (B) est mesurée de telle manière que l'aptitude à la prolifération des lymphocytes T, qui sont contenus dans les concentrats de thrombocytes, est diminuée d'au moins 50%, de préférence de plus de 75%.

6. Procédé selon l'une quelconque des revendications précédentes, **caractérisé en ce que**, dans l'étape (B), l'introduction d'énergie est de 0,5 à 3 J/cm².

7. Procédé selon l'une quelconque des revendications précédentes, **caractérisé en ce que**, dans l'étape (B), la suspension est soumise à un rayonnement d'une plage de longueurs d'onde de 290 à 320 nm.

8. Procédé selon l'une quelconque des revendications précédentes, **caractérisé en ce que** les suspensions de thrombocytes sont des concentrats de thrombocytes.

9. Procédé selon l'une quelconque des revendications précédentes, **caractérisé en ce que** les suspensions de thrombocytes ou, selon le cas, les concentrats de thrombocytes sont obtenus à partir de dons de sang ou par thrombocytaphérèse.

10. Procédé selon l'une quelconque des revendications précédentes, **caractérisé en ce que** le traitement de la suspension selon l'étape (A) et/ou l'étape (B) est réalisé dans des récipients en matériau synthétique transparent à chaque rayonnement.

11. Procédé selon l'une quelconque des revendications précédentes, **caractérisé en ce que** le traitement de la suspension selon l'étape (A) et/ou l'étape (B) est réalisé dans un appareillage de débit transparent à chaque rayonnement.

12. Procédé selon l'une quelconque des revendications précédentes, **caractérisé en ce que** la concentration en virus de la suspension est diminuée d'au moins 5, de préférence d'au moins 6 échelons log₁₀.
